# EUROPEAN PATENT SPECIFICATION

(11) **EP 1 579 224 B1**
(45) Date of publication and mention of the grant of the patent: **17.08.2016**
(21) Application number: 03814967.0
(22) Date of filing: 24.12.2003
(51) Int. Cl.: G01R 1/00

(54) **IMPLANTABLE MARKER WITH A WIRELESS SIGNAL TRANSMITTER COMPATIBLE FOR USE IN MAGNETIC RESONANCE IMAGING DEVICES AND/OR SUITABLE FOR USE IN RADIATION IMAGING PROCESSES**
IMPLANTIERBARE MARKIERUNG MIT EINEM DRAHTLOSEN SIGNALSENDER,KOMPATIBEL ZUR VERWENDUNG IN MAGNETRESONANZABBILDUNGSEINRICHTUNGEN UND/ODERGEEIGNET FÜR DIE VERWENDUNG IN STRAHLUNGSABBILDUNGSPROZESSEN
MARQUEUR IMPLANTABLE AVEC UN EMETTEUR DE SIGNAUX SANS FIL D'UTILISATION COMPATIBLE DANS DES DISPOSITIFS D'IMAGERIE PAR RESONANCE MAGNETIQUE ET/OU APTE A ETRE UTILISE DANS DES PROCEDES D'IMAGERIE PAR RAYONNEMENT

(30) Priority: 30.12.2002 US 334698
(43) Date of publication of application: 28.09.2005
(73) Proprietor: Varian Medical Systems, Inc., Palo Alto, CA 94304 (US)
(72) Inventor: DIMMER, Steven, C., Bellevue, WA 98005 (US); HADFORD, Eric, Snohomish, WA 98296 (US)
(74) Representative: Hoarton, Lloyd Douglas Charles
(86) International application number: PCT/US2003/041329
(87) International publication number: WO 2004/061460

(56) References cited:
- WO-A-02/39917
- WO-A-03/024329
- WO-A1-98/30166
- US-A- 6 026 818
- US-A1- 2002 193 685
- US-B2- 6 812 842
- STRADIOTTI P ET AL: "Metal-related artifacts in instrumented spine. Techniques for reducing artifacts in CT and MRI: state of the art", EUROPEAN SPINE JOURNAL, SPRINGER, BERLIN, DE, vol. 18, no. 1, 13 May 2009 (2009-05-13), pages 102-108, XP019720103, ISSN: 1432-0932

## Description

### TECHNICAL FIELD

The present invention is directed toward markers with signal transmitters that wirelessly transmit location signals. The markers are compatible for use in magnetic resonance devices and/or suitable for use in radiation imaging processes. Several embodiments of the markers are permanently implantable or semi-permanently implantable in patients for locating at least one target in and/or on the patient.

### BACKGROUND

Medical procedures often require locating and treating target areas within a patient. Radiation therapy and many surgical procedures require locating the target with a high degree of precision to limit collateral damage to healthy tissue around the target. It is particularly important to know or estimate the precise location of the target in radiation oncology because it is (a) desirable to accurately determine the accumulated dosage applied to the target and (b) detrimental to expose adjacent body parts to the radiation. In applications for treating prostate cancer, for example, it is detrimental to irradiate the colon, bladder or other neighboring body parts with the high-intensity radiation beam. Surgical applications, such as breast surgery and other procedures involving soft tissue, also require knowing the precise location of a target because a lesion in soft tissue is not necessarily fixed relative to external landmarks on the patient.

Many imaging systems have been used to locate areas or particular targets in a patient before performing radiation oncology or surgical procedures. Although x-ray, Magnetic Resonance Imaging (MRI), CT and other imaging techniques are useful to locate targets within the body at a pre-operative stage of a procedure, they are often not suitable or difficult to use in real time during surgery or radiation therapy. For example, the location of a lesion in soft tissue or in an organ may shift relative to external landmarks on the patient between the pre-operative imaging procedure and the actual radiation or surgical procedure. Additionally, when imaging systems are used during a radiation or surgical procedure, they may not provide sufficiently accurate measurements of the location of the lesions and they may interfere with the radiation or surgical procedure. Therefore, imaging techniques by themselves are generally not well suited for accurately identifying the actual location of a target for many medical applications.

Another technique to locate a target in a patient is to implant a marker relative to the target. Several types of tags or markers with resonating magnetic circuits have been developed to track feeding tubes, tag items, and mark tissue. For example, implantable markers that generate a signal have been proposed for use to locate a selected target in a patient in radiation oncology procedures. U. S. Patent No. 6,385, 482 B1 issued to Boksberger et al. discloses a device having an implanted emitter unit located inside or as close as possible to a target object, and a plurality of receiver units that are located outside of the patient. Boksberger discloses determining the location of the target object by energizing the emitter unit using a generator and sensing the signal from the emitter unit with the receiver units. Boksberger discloses and claims that the receiver units are configured to determine the gradient of the magnetic field generated by the emitter unit.

Boksberger further discloses that the emitter unit is energized using wired connection to the external generator. Boksberger also indicates that it is conceivable to use an emitter unit that is energized by a battery or excited by an electromagnetic field generated by the external generator. The wired device disclosed in Boksberger, however, may not be suitable for use in radiation oncology and many surgical procedures because it is impractical to leave a wired marker implanted in a patient for the period of time of such procedures (e. g. five to forty days). Moreover, Boksberger does not disclose or suggest anything with respect to providing an implantable emitter unit that is (a) suitable for use in radiation imaging processes or (b) compatible for use in magnetic resonance imaging devices after being implanted in a patient.

WO 03/024329 is a prior art document falling under Art.54(3) EPC and discloses a miniature resonating marker assembly that includes, in one embodiment, a ferromagnetic core, a wire coil disposed around the core, and a capacitor connected to the wire coil adjacent to the magnetic core. The core, coil, and capacitor form a signal element that, when energized, generates a magnetic field at a selected resonant frequency. The magnetic field has a magnetic center point positioned along at least one axis of the signal element. An inert encapsulation member encapsulates the signal element therein and defines a geometric shape of the resonating marker assembly. The geometric shape has a geometric center point substantially coincident with the magnetic center point along at least a first axis of the signal element. The shape and configuration of the assembly also provides for a miniature signal element specifically tuned to resonate at a selected frequency with a high quality factor.

US 2002/193685 discloses a system and method for accurately locating and tracking the position of a target, such as a tumor or the like, within a body. In one embodiment, the system is a target locating and monitoring system usable with a radiation delivery source that delivers selected doses of radiation to a target in a body. The system includes one or more excitable markers positionable in or near the target, an external excitation source that remotely excites the markers to produce an identifiable signal, and a plurality of sensors spaced apart in a known geometry relative to each other. Paragraph [0041] of US'685 discloses a "resonating marker 31 having a ferrite core 46 wrapped by a conductive winding 48, and the winding is connected to a small capacitor 50."

WO 02/39917 discloses systems and methods for locating and defining a target(M) location within a human body. The system can include at least one marker (30,_1100), a probe (1200), and a detector (34) for use in locating the markers (30, 1100) by providing information to a surgeon that is representative of changes in proximity between the probe (1200) and the marker (30, 1100). The marker (30,1100) can have various detection characteristics, e.g., gamma radiation, that are detectable by an associated probe (1200) and detector (34).The tissue volume (T) is removed by manipulating a cutting tool based on the proximity information provided by the detector (34), which can be used by the surgeon to define the boundary of the tissue volume (T).

Another technique to locate a target in a patient is to implant passive, gold fiducials in or near the target site. The positions of the gold fiducials are determined periodically using radiation. Although gold fiducials are useful for localizing a target within a patient, these systems do not provide sufficiently accurate real time measurements of the target site location during radiation oncology procedures.

Other types of tags or markers with resonating magnetic circuits have been developed. These markers have been used to tag sponges and other items used during surgery or locate the general location of feeding tubes or other instruments in other procedures. One significant challenge of miniature, wireless markers is to provide a sufficiently strong signal to be accurately detected by sensors outside of the body.

Additionally, a challenge of using markers with resonating magnetic circuits is determining the relative location between the marker and the target so that the target can be tracked during a procedure or therapy. Accurately determining the location of the marker relative to the target is a precondition for accurately tracking the target based on the resonating magnetic field,generated by the implanted marker. One reason that it is difficult to accurately determine the location of the marker relative to the target is that it can be difficult to identify magnetic resonating markers in radiographic images. The markers are difficult to see in radiographic images because (a) they should be very small so that they may be implanted for an extended period of time, and (b) they may not be sufficiently visible in high voltage radiation applications (i. e., megavolt radiation imaging). Moreover, even when a magnetic marker can be identified in an image, it can still be challenging to determine the orientation of the magnetic field generated by the marker relative to the target because it is often difficult to determine the orientation of the marker in the image.

As such, implantable markers with resonating magnetic circuits may be difficult to use in radiation therapies and surgical procedures that require highly accurate localization of the target.

### BRIEF DESCRIPTION OF THE DRAWINGS

Figure 1 is an isometric view of an implantable wireless marker in accordance with an embodiment of the invention with a section cut away to illustrate internal components.
Figure 2 is a cross-sectional view taken along a longitudinal axis of an embodiment of the marker of Figure 1.
Figure 3 is a cross-sectional view in a plane normal to a longitudinal axis of a marker in accordance with an embodiment of the marker shown in Figure 1.
Figure 4 is a cross-sectional view taken along a longitudinal axis of a marker in accordance with an embodiment of the invention after being implanted in a patient.
Figure 5 is a diagram of a display of a magnetic resonance image with an artifact by a magnetic marker.
Figure 6 is a cross-sectional view taken along a longitudinal axis of a marker in accordance with another embodiment of the invention.
Figure 7A is an isometric view of a wireless marker in accordance with an embodiment of the invention with a section cut away to illustrate internal components.
Figure 7B is a cross-sectional view of the wireless marker of Figure 7A taken along line 7B-7B.
Figure 7C is an illustration of a radiographic image of the marker of Figures 7A-B.
Figure 8A is an isometric view of a wireless marker in accordance with another embodiment of the invention.
Figure 8B is a cross-sectional view of the wireless marker of Figure 8A taken along line 8B-8B.
Figure 9A is an isometric view of a wireless marker in accordance with another embodiment of the invention.
Figure 9B is a cross-sectional view of the wireless marker of Figure 9A taken along line 9B-9B.
Figure 10 is an isometric view of a wireless marker in accordance with yet another embodiment of the invention with a section cut away to illustrate internal components.
Figure 11 is an isometric view of a wireless marker in accordance with still another embodiment of the invention with a section cut away to illustrate internal components.

### DETAILED DESCRIPTION

### A. Overview

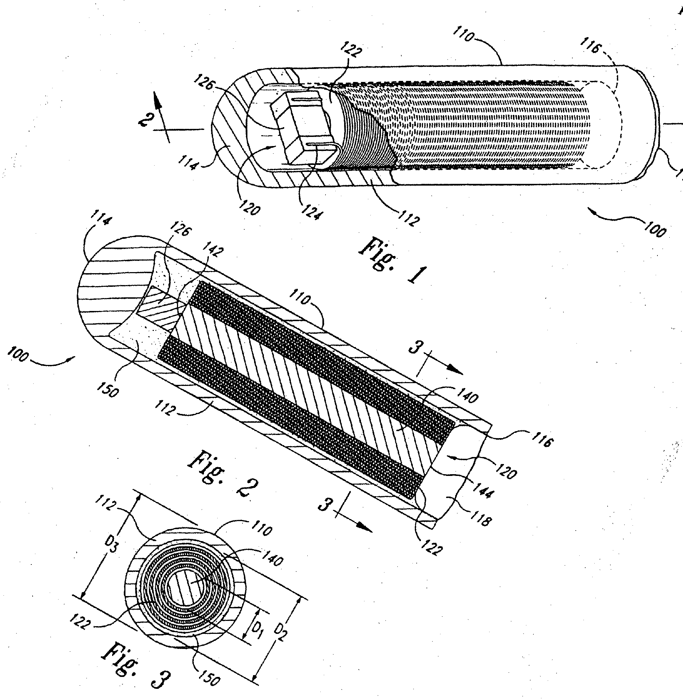

The following disclosure describes several embodiments of wireless markers configured to be attached to a patient either by being implanted into the patient or adhered externally to the skin of the patient. Several embodiments of the markers are highly suitable for use in radiographic imaging systems and other types of imaging systems to determine the location and orientation of the magnetic field with respect to the target of the patient. Other embodiments of the markers are compatible for use in powerful magnetic fields generated by magnetic resonance imaging devices either in addition to or in lieu of being suitable for use in radiographic imaging systems. Several embodiments and features of markers in accordance with the invention are set forth and described in Figures 1-11. It will be appreciated that other embodiments of markers in accordance with the invention can include additional or different features than those shown in Figures 1-11. Additionally, it will be appreciated that several embodiments of markers in accordance with the invention do not include all of the features shown in these figures. For purposes of brevity, like reference numbers refer to similar or identical components.

The present invention provides a leadless implantable marker, a method of tracking a target and a method of manufacturing a marker as claimed.

Some embodiments of wireless markers can be suitable for radiographic imaging in addition to or in lieu of being compatible with magnetic resonance imaging equipment. For example, one embodiment of a wireless marker for localizing a target of a patient comprises a casing and a magnetic transponder at least partially received in the casing. The magnetic transponder produces a wirelessly transmitted magnetic field in response to a wirelessly transmitted excitation energy. The magnetic transponder also has a magnetic centroid. The marker also comprises an imaging element carried by the casing and/or the magnetic transponder. The imaging element has a radiographic profile in a radiographic image such that the marker has a radiographic centroid at least approximately coincident with the magnetic centroid.

The imaging element can have several different configurations and be composed of many different materials. For example, to be visible on megavoltage x-ray images, the imaging element can comprise a single contrast element or a plurality of contrast elements composed of a high density material and having a sufficient thickness and cross-sectional area to absorb a substantial fraction of photons incident on the imaging element. The image is formed by the reduction of photon flux density in the path from the x-ray source through the imaging element to a radiographic imaging device or film. In other applications that use lower acceleration voltages for the imaging radiation, the imaging element can be a contrast element having a lower density or a different configuration that is not suitable for use with megavoltage x-ray images.

### B. Examples of Markers for Use In MRI Procedures

Figure 1 is an isometric view of an implantable marker 100 in accordance with an embodiment of the invention with a portion cut away to illustrate internal components. The embodiment of the marker 100 shown in Figure 1 includes a casing 110 and a magnetic transponder 120 (e.g., a resonating circuit) in the casing 110. The terms magnetic transponder 120 and resonating circuit 120 are used interchangeably throughout. The casing 110 is a biocompatible barrier configured to be implanted in the patient or otherwise attached to the patient. The casing 110 can be a generally cylindrical capsule that is sized to fit within a 14 gauge needle for percutaneous implantation, but the casing can have other configurations and be larger or smaller. The casing 110, for example, can have barbs to anchor the casing 110 in soft tissue or an adhesive for attaching the casing 110 externally to the skin of a patient. Suitable anchoring devices are disclosed in International Publication No. WO 02/39917 A1. In one embodiment, the casing 110 includes (a) a glass capsule or shell 112 having a closed end 114 and an open end 116, and (b) a sealant 118 in the open end 116 of the shell 112. The casing 110 and sealant 118 can be made from plastics, ceramics, glass or other suitable biocompatible materials.

The resonating circuit 120 produces a wirelessly transmitted signal in response to a wirelessly transmitted excitation signal. In one embodiment, the resonating circuit 120 comprises a coil 122 defined by a plurality of windings of a conductor 124. Many embodiments of the resonating circuit 120 also include a capacitor 126 coupled to the coil 122. The coil 122 resonates at a selected resonant frequency. The coil 122 can resonate at the selected resonant frequency solely using the parasitic capacitance of the windings without having a capacitor, or the selected resonant frequency can be produced using the combination of the coil 122 and the capacitor 126. The coil 122 by itself or in combination with the capacitor 126 accordingly defines a signal transmitter that generates an alternating magnetic field at the selected resonant frequency in response to the excitation signal. The conductor 124 of the illustrated embodiment can be hot air or alcohol bonded wire having a gauge of approximately 45-52. The coil 122 can have 800-2000 turns. The windings are preferably wound in a tightly layered coil.

The resonating circuit 120 is powered by a wirelessly transmitted excitation signal such that the resonating circuit is leadless, i. e. , not connected to external lead wires which extend through or project from the casing 110. In one embodiment, the resonating circuit 120 can be energized by an alternating excitation magnetic field generated externally with respect to the patient at the resonant frequency of the resonating circuit. In response to the excitation field, the resonating circuit 120 produces a marker signal or response signal that can be measured by a sensor array positioned externally with respect to the patient.

Suitable devices for generating the magnetic excitation field and sensing the marker signal are disclosed in U. S. Patent Publication Nos. US2003122653; US2003117269; and US2003117270.

Figure 2 is a cross-sectional view of an embodiment of the marker 100 taken along a longitudinal axis 2-2 shown in Figure 1. The marker 100 further includes a ferromagnetic element 140 having a first end 142 and a second end 144. The ferromagnetic element 140 is at least partially surrounded by the coil 122. In the particular embodiment shown in Figure 2, the coil 122 surrounds the ferromagnetic element 140 from the first end 142 to the second end 144. In other embodiments, the coil 122 surrounds only a portion of the ferromagnetic element 140. The capacitor 126 can be positioned at the first end 142 of the ferromagnetic element 140. Additionally, the resonating circuit 120 and the ferromagnetic element 140 can be fixed to the casing 110 by an adhesive 150.

The ferromagnetic element 140 is preferably composed of ferrite or other materials that have high magnetic permeability compared to free space. The amount of energy that the inductor is capable of storing is limited, in part, by the magnetic field saturation of the ferromagnetic element 140. To store more energy in a miniature wireless marker, the prior art taught that the size of the ferromagnetic material should be maximized within the limited space of the marker. As shown in Figure 2, however, the volume of the ferromagnetic element 140 is significantly less than the available volume within the casing 110. The smaller volume of the ferromagnetic element 140 reduces the force exerted on the marker 100 when the marker 100 is placed in a magnetic resonance imaging device having a magnetic field strength of 1.5 T with a corresponding gradient field of approximately 3 T/m.

In one embodiment, the ferromagnetic element has a volume such that when the marker is in a magnetic resonance device, then the force exerted on the marker by the magnetic field is less than gravitational force exerted on the marker. Additionally, the small volume of the ferromagnetic element 140 reduces the size of the artifact in an image from a magnetic resonance device. It will be appreciated that ferromagrietic materials will produce an artifact (i. e. , a region in which image information is suppressed) in an image produced by a magnetic resonance imaging device. The volume of the ferromagnetic element 140 can be reduced to a size such that it produces a small artifact in an image from a magnetic resonance device. In general, such ferromagnetic elements 140 have small diameters less than the size of commercially available ferrite rods for transponder applications, which are as small as 0.75mm in diameter (i. e. , ferrite rods available from Ferroxcube of Spain).

Figure 3 is a cross-sectional view of the marker 100 taken along line 3-3 of Figure 2.

In one example, the ferromagnetic element 140 is a ferrite rod having a diameter D, of approximately 0.20-0. 70 mm, but the ferromagnetic element 140 can have other cross-sectional configurations. For example, an extruded ferrite rod can have an elliptical, oval or polygonal cross section. The ferromagnetic element 140 can have a length of approximately 2.0-20 mm. In one particular example the ferromagnetic element 140 has a diameter of approximately 0.25-0. 50 mm and a length of 2-12 mm. In embodiments of the invention, the ferromagnetic element 140 has a diameter of 0.30-0. 35 mm and a length of 4.0-6. 0 mm. In some examples, the coil 122 has an inner diameter of approximately 0.20-0.80 mm and an outer diameter D2 of approximately 0.6-1. 4mm or 0.8-1.9 mm. The casing 110 can have an outer diameter D3 of approximately 1.0-3. 0 mm. In other examples, the coil 122 can have different inner and outer diameters, and the casing 110 can have a different outer diameter. In another particular example, the diameter D, of the ferromagnetic element 140 is approximately 0.30-0. 50 mm, the inner diameter of the coil 122 is approximately 0.30-0. 60 mm, the outer diameter DZ of the coil 122 is approximately 1.2-1. 9 mm (or 1.2-1.4 mm), and the outer diameter D3 of the casing 110 is approximately 1.8-2. 0 mm. The volume of the ferromagnetic element 140 can be approximately 0.5-19. 0 mm³.

The marker 100 is constructed by manufacturing the ferromagnetic element 140, placing the coil 122 around the ferromagnetic element 140, and encapsulating the resonating circuit 120 and the ferromagnetic element 140 in the casing 110. The ferromagnetic element 140 can be manufactured using extrusion, coring, or high pressure molding processes to form a ferrite rod having a diameter of approximately 0.2-0. 7 mm.

The coil 122 is formed by winding the conductor 124 around either the ferromagnetic element 140, a sleeve around the ferromagnetic element 140, or a mandrel separate from the ferromagnetic element 140. In one embodiment, the conductor 124 is wrapped directly onto the ferromagnetic element 140, but this may not be feasible in many applications because it may break ferromagnetic elements having a diameter less than 0.5 mm. In another embodiment, a retractable sleeve can slide along the ferromagnetic element 140 as the conductor 124 is wound directly onto the ferromagnetic element. The sleeve is expected to support the ferromagnetic element 140 as the first layer of turns are wrapped around the ferromagnetic element 140. The first layer of turns supports the rod so that subsequent layers of turns can be wound onto the first layer. In still another embodiment, the coil 122 is wound around a mandrel separately from the ferromagnetic element 140.

The coil 122 is then removed from the mandrel and the ferromagnetic element 140 is inserted into the inner diameter of the coil 122. This embodiment can result in a small gap between the ferromagnetic element 140 and the inner diameter of the coil 122. This gap should be minimized in optimal circumstances to increase the performance of the resonating circuit 120. After the ferromagnetic element 140 is positioned within the coil 122, this assembly is adhered to the casing 110 using the adhesive 150, and the sealant 118 is used to close the open end 116 of the casing 110.

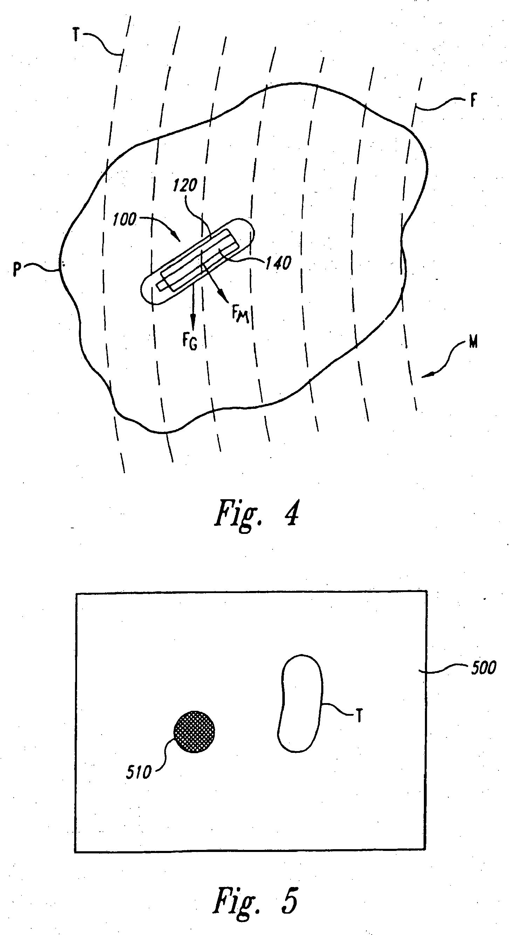

Figure 4 is a representative view of the operation of the marker 100 in an magnetic field M generated by a magnetic resonance imaging device (not shown). The magnetic field M is an imaging magnetic field. In this embodiment, a patient is placed in a magnetic resonance imaging device to image a portion P of the patient. The imaging magnetic field M includes a plurality of flux lines F. Because the ferromagnetic element 140 has a high magnetic permeability, the ferromagnetic element 140 exerts a magnetic force FM in the presence of the magnetic field M due to the presence of DC and gradient magnetic fields.

The magnitude of the magnetic force FM is a function of the volume and the type of material (i. e. , magnetic saturation) of the ferromagnetic element 140. The volume of the ferromagnetic element 140 is selected so that the magnetic force FM caused by the interaction between the ferromagnetic element 140 and the magnetic field M is less than the gravitational force FG exerted against the marker 100. This will ensure that the magnetic field M does not cause the marker 100 to move within the portion P of the patient any more than the force of gravity will cause movement of the marker 100.

Figure 5 is a schematic representation of a magnetic resonance image 500 that shows a target location T within a body part of a patient. The image 500 includes an artifact 510 caused by the ferromagnetic element 140 of the marker 100. The artifact 510 is typically much larger than the size of the marker, and thus it tends to obscure the actual location of the marker and the images of tissue adjacent to the marker. The size of the artifact 510 is related to the size of the ferromagnetic element 140 in the marker 100. In several examples, the volume of the ferromagnetic element 140 is selected to produce an artifact not greater than 1,500 mm² in an image produced by a resonance imaging device field having a DC field strength of 1.5 T. In other embodiments, the volume of the ferromagnetic element 140 is selected to produce an artifact not greater than 400-1,200 mm², and in other cases not greater than 400-800 mm² in an image produced by a magnetic resonance imaging device field having a DC field strength of 1.5 T.

### C. Examples of Markers with Enhanced Radiographic Properties

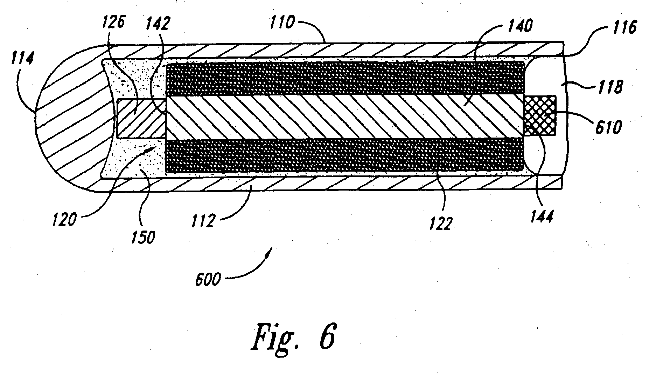

Figure 6 is a cross-sectional view of a marker 600 in accordance with another embodiment of the invention. The marker 600 is substantially similar to the marker 100 shown in Figure 2, but the marker 600 further includes a module 610 at the second end 144 of the ferromagnetic element 140. The module 610 is preferably configured to be symmetrical with respect to the capacitor 126 at the first end 142 of the ferromagnetic element 140. The module 610, more specifically, is configured to produce a similar radiographic image as the capacitor 126 in an x-ray. In one embodiment, the module 610 is configured such that the magnetic centroid of the marker is at least substantially coincident with the radiographic centroid of the marker. In other embodiments that use CT or other types of imaging modalities, the module 610 is configured to produce a symmetrical image relative to the capacitor 126. For example, the module 610 can be another capacitor identical to the capacitor 126 that may or may not be electrically coupled to the coil 122. In other embodiments, the module 610 can be an electrically inactive element that is not electrically connected to the resonating circuit 120 or another type of electrically active element that is electrically coupled to the resonating circuit 120. Suitable electrically inactive modules include ceramic blocks shaped like the capacitor 126. In either case, one purpose of the module 610 is to have the same characteristics as the electrically active capacitor 126 in x-ray, CT, and other imaging techniques. Since the markers may be located via radiographic methods (e. g. , CT, or x-ray) to determine the marker centroid positions relative the target tissue prior to therapy, an error in the position of the marker radiographic and magnetic centroids may result in a fixed positional error during therapy.

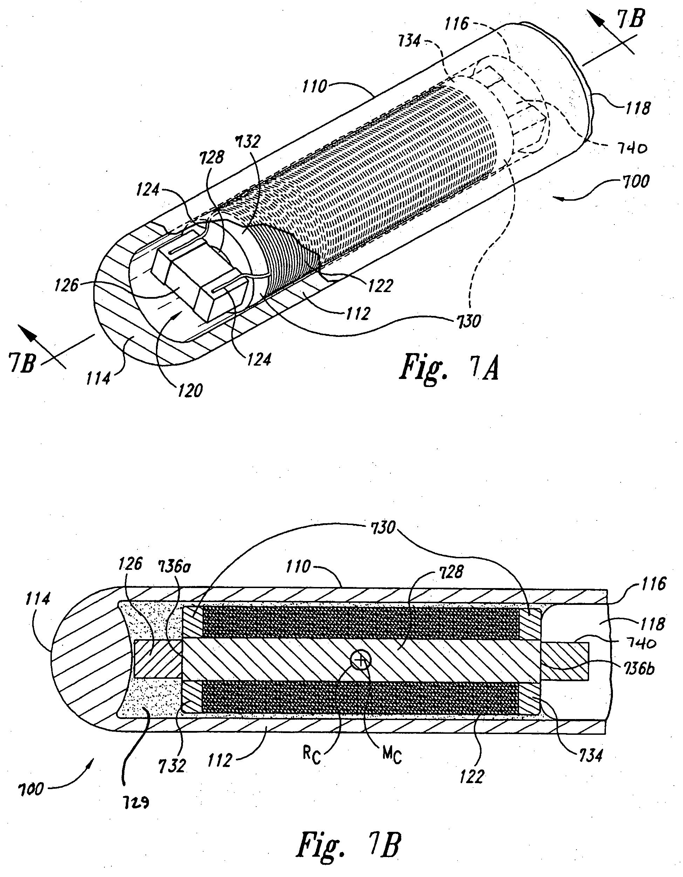

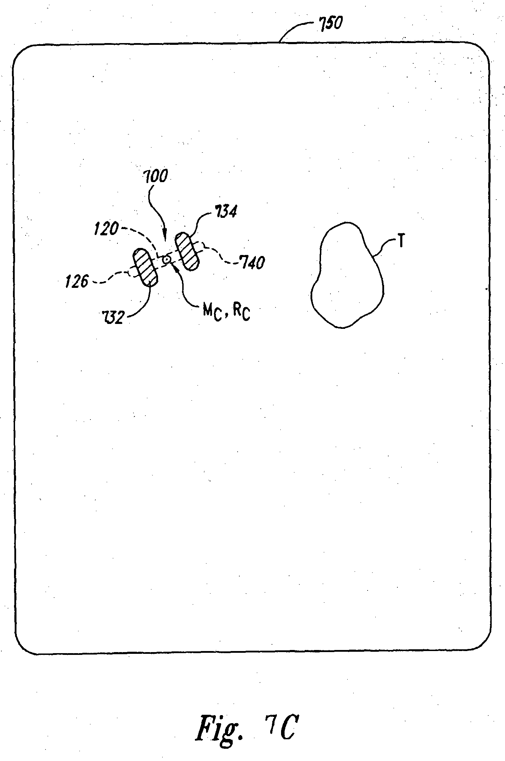

Figure 7A is an isometric view of a marker 700 in accordance with an embodiment of the invention with a portion cut away to illustrate internal components. The marker 700, shown in Figure 7A is similar to the marker 100 shown in Figure 1 or the marker 600 shown in Figure 6, and like reference numbers refer to like components. As such, the embodiment of the marker 700 shown in Figure 7 includes a casing 110 and a magnetic transponder 120 in the casing 110. The magnetic transponder 120 can be a resonating circuit that produces a wirelessly transmitted signal in response to a wirelessly transmitted excitation field. The magnetic transponder 120 can accordingly comprise the coil 122, the capacitor 126, and a core 728. The core 728 can be a ferromagnetic element that is configured to be compatible in MRI devices as set forth above with reference to Figures 1-6, but the core 728 need not be MRI compatible. As such, the core 728 does not necessarily have the same dimensions as the ferromagnetic element 140 described above in Figures 1-6.

The marker 700 also includes an imaging element that enhances the radiographic image of the marker to make the marker more discernible in radiographic images. The imaging element also produces a radiographic profile in a radiographic image such that the marker has a radiographic centroid at least approximately coincident with the magnetic centroid of the magnetic transponder 120. As explained in more detail below, the radiographic and magnetic centroids do not need to be exactly coincident with each other, but rather can be within an acceptable range.

Figure 7B is a cross-sectional view of the marker 700 along line 7B-7B that illustrates an adhesive 729 to adhere the magnetic transponder 120 to the casing 110 and an imaging element 730 in accordance with an embodiment of the invention. The imaging element 730 illustrated in Figures 7A-B includes a first contrast element 732 and second contrast element 734. The first and second contrast elements 732/734 are generally configured with respect to the magnetic transponder 120 so that the marker 700 has a radiographic centroid Rc that is at least substantially coincident with the magnetic centroid Me of the magnetic transponder 120. For example, when the imaging element 730 includes two contrast elements, the contrast elements can be arranged symmetrically with respect to the magnetic transponder 120 and/or each other. The contrast elements can also be radiographically distinct from the magnetic transponder 120. In such an embodiment, the symmetrical arrangement of distinct contrast elements enhances the ability to accurately determine the radiographic centroid of the marker 700 in a radiographic image.

The first and second contrast elements 732/734 illustrated in Figures 7A-B are continuous rings positioned at opposing ends of the core 728. The first contrast element 732 can be at or around a first end 736a of the core 728, and the second contrast element 734 can be at or around a second end 736b of the core 728. The continuous rings shown in Figures 7A-B have substantially the same diameter and thickness. The first and second contrast elements 732/734, however, can have other configurations and/or be in other locations relative to the core 728 in other embodiments. For example, the first and second contrast elements 732/734 can be rings with different diameters and/or thicknesses.

The radiographic centroid of the image produced by the imaging element 730 does not need to be absolutely coincident with the magnetic centroid Mc, but rather the radiographic centroid and the magnetic centroid should be within an acceptable range. For example, the radiographic centroid Rc can be considered to be at least approximately coincident with the magnetic centroid Me when the offset between the centroids is less than approximately 5 mm. In more stringent applications, the magnetic centroid Me and the radiographic centroid Rc are considered to be at least substantially coincident with each other when the offset between the centroids is 2 mm or less. In other applications, the magnetic centroid Me is at least approximately coincident with the radiographic centroid Rc when the centroids are spaced apart by a distance not greater than half the length of the magnetic transponder 120 and/or the marker 700.

The imaging element 730 can be made from a material and configured appropriately to absorb a high fraction of incident photons of a radiation beam used for producing the radiographic image. For example, when the imaging radiation has high acceleration voltages in the megavoltage range, the imaging element 730 is made from, at least in part, high density materials with sufficient thickness and cross-sectional area to absorb enough of the photon fluence incident on the imaging element to be visible in the resulting radiograph. Many high energy beams used for therapy have acceleration voltages of 6 MV - 25 MV, and these beams are often used to produce radiographic images in the 5 MV-10 MV range, or more specifically in the 6 MV-8 MV range. As such, the imaging element 730 can be made from a material that is sufficiently absorbent of incident photon fluence to be visible in an image produced using an beam with an acceleration voltage of 5 MV-10 MV, or more specifically an acceleration voltage of 6 MV-8 MV.

Several specific examples of imaging elements 730 can be made from gold, tungsten, platinum and/or other high density metals. In these examples the imaging element 730 can be composed of materials having a density of 19.25 g/cm³ (density of tungsten) and/or a density of approximately 21.4 g/cm³ (density of platinum). Many embodiments of the imaging element 730 accordingly have a density not less than 19 g/cm³. In other embodiments, however, the material (s) of the imaging element 730 can have a substantially lower density. For example, imaging elements with lower density materials are suitable for applications that use lower energy radiation to produce radiographic images. Moreover, the first and second contrast elements 732/734 can be composed of different materials such that the first contrast element 732 can be made from a first material and the second contrast element 734 can be made from a second material.

Referring to Figure 7B, the marker 700 can further include a module 740 at an opposite end of the core 728 from the capacitor 126. In the embodiment of the marker 700 shown in Figure 7B, the module 740 is configured to be symmetrical with respect to the capacitor 126 to enhance the symmetry of the radiographic image. As with the first and second contrast elements 732/734, the module 740 and the capacitor 126 are arranged such that the magnetic centroid of the marker is at least approximately coincident with the radiographic centroid of the marker 700. The module 740 can be another capacitor that is identical to the capacitor 126, or the module 740 can be an electrically inactive element.

Suitable electrically inactive modules include ceramic blocks shaped like the capacitor 126 and located with respect to the coil 122, the core 728 and the imaging element 730 to be symmetrical with each other. In still other embodiments the module 740 can be a different type of electrically active element electrically coupled to the magnetic transponder 120.

The module 740 can accordingly perform much the same function and be constructed in much the same manner as the module 610 described above.

One specific process of using the marker involves imaging the marker using a first modality and then tracking the target of the patient and/or the marker using a second modality. For example, the location of the marker relative to the target can be determined by imaging the marker and the target using radiation. The marker and/or the target can then be localized and tracked using the magnetic field generated by the marker in response to an excitation energy. Suitable applications for such bi-modal use of the marker 700 and suitable systems for localizing/tracking the marker are disclosed and described in the following pending U. S. Publication Nos.: US2003192557; US2004125916; US2002193685; US2003052785; US2003117270; US2004176931; and US2004123871.

The marker 700 shown in Figures 7A-B is expected to provide an enhanced radiographic image compared to conventional magnetic markers for more accurately determining the relative position between the marker and the target of a patient. Figure 7C, for example, illustrates a radiographic image 750 of the marker 700 and a target T of the patient. The first and second contrast elements 732/734 are expected to be more distinct in the radiographic image 750 because they can be composed of higher density materials than the components of the magnetic transponder 120. The first and second contrast elements 732/734 can accordingly appear as bulbous ends of a dumb-bell shape in applications in which the components of the magnetic transponder 120 are visible in the image. In certain megavolt applications, the components of the magnetic transponder 120 may not appear at all on the radiographic image 750 such that the first and second contrast elements 732/734 will appear as distinct regions that are separate from each other. In either embodiment, the first and second contrast elements 732/734 provide a reference frame in which the radiographic centroid Rc of the marker 700 can be located in the image 750. Moreover, because the imaging element 730 is configured so that the radiographic centroid Rc is at least approximately coincident with the magnetic centroid Me, the relative offset or position between the target T and the magnetic centroid Me can be accurately determined using the marker 700. The embodiment of the marker 700 illustrated in Figures 7A-C, therefore, is expected to mitigate errors caused by incorrectly estimating the radiographic and magnetic centroids of markers in radiographic images.

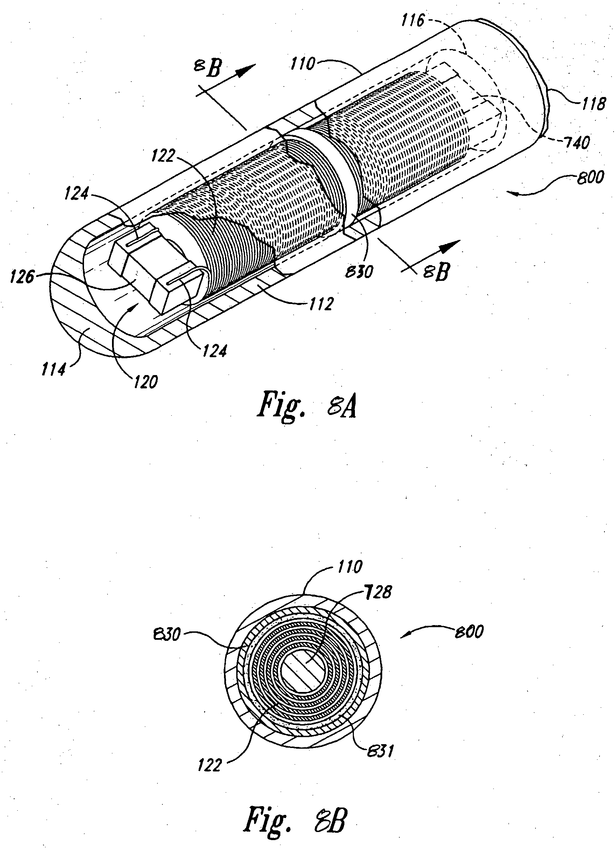

Figure 8A is an isometric view of a marker 800 with a cut away portion to illustrate internal components, and Figure 8B is a cross-sectional view of the marker 800 taken along line 8B-8B of Figure 8A. The marker 800 is similar to the marker 700 shown above in Figure 7A, and thus like reference numbers refer to like components. The marker 800 differs from the marker 700 in that the marker 800 includes an imaging element 830 having a single contrast element. The imaging element 830 is generally configured relative to the magnetic transponder 120 so that the radiographic centroid of the marker 800 is at least approximately coincident with the magnetic centroid of the magnetic transponder 120. The imaging element 830, more specifically, is a ring extending around the coil 122 at a medial region of the magnetic transponder 120. The imaging element 830 can be composed of the same materials described above with respect to the imaging element 730 in Figures 7A- B. The imaging element 830 can have an inner diameter that is approximately equal to the outer diameter of the coil 122, and an outer diameter within the casing 110. As shown in Figure 8B, however, a spacer 831 can be between the inner diameter of the imaging element 830 and the outer diameter of the coil 122.

The marker 800 is expected to operate in a manner similar to the marker 700 described above. The marker 800, however, does not have two separate contrast elements that provide two distinct, separate regions in a radiographic image. The imaging element 830 is still highly useful in that it identifies the radiographic centroid of the marker 800 in a radiographic image, and it can be configured so that the radiographic centroid of the marker 800 is at least approximately coincident with the magnetic centroid of the magnetic transponder 120.

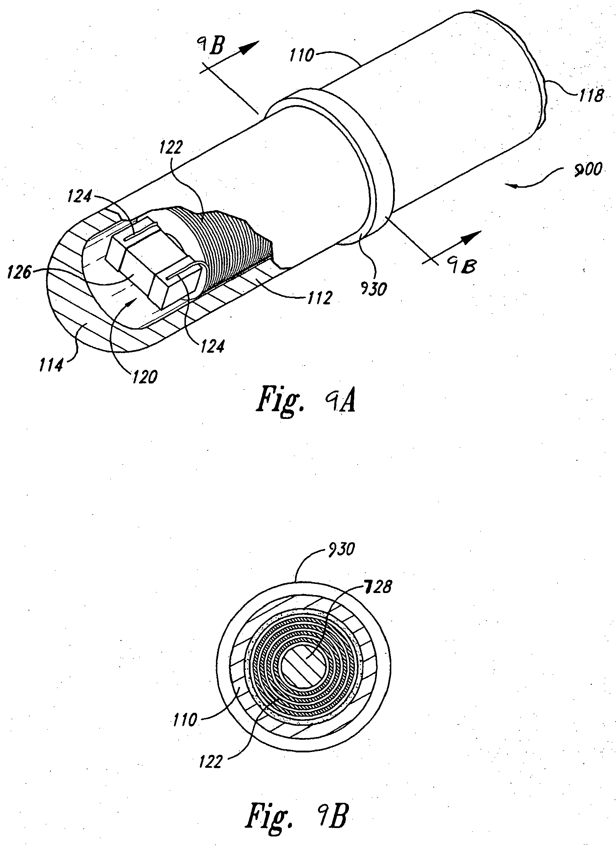

Figure 9A is an isometric view of a marker 900 having a cut away portion, and Figure 9B is a cross-sectional view of the marker 900 taken along line 9B-9B. The marker 900 is substantially similar to the marker 800 shown in Figures 8A-B, and thus like reference numbers refer to like components in Figures 7A-9B. The imaging element 930 can be a high density ring configured relative to the magnetic transponder 120 so that the radiographic centroid of the marker 900 is at least approximately coincident with the magnetic centroid of the magnetic transponder 120. The marker 900, more specifically, includes an imaging element 930 around the casing 110. The marker 900 is expected to operate in much the same manner as the marker 800 shown in Figures 8A-B.

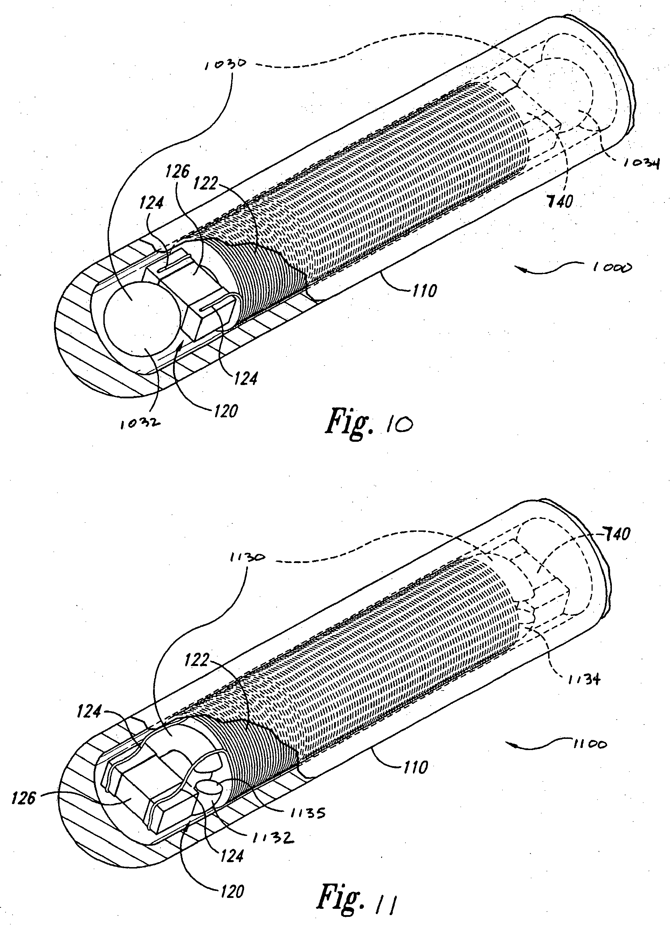

Figure 10 is an isometric view with a cut away portion illustrating a marker 1000 in accordance with another embodiment of the invention. The marker 1000 is similar to the marker 700 shown in Figures 7A-C, and thus like reference numbers refer to like components in these Figures. The marker 1000 has an imaging element 1030 including a first contrast element 1032 at one end of the magnetic transponder 120 and a second contrast element 1034 at another end of the magnetic transponder 120. The first and second contrast elements 1032/1034 are spheres composed of a suitable high density material (s). The contrast elements 1032/1034, for example, can be composed of gold, tungsten, platinum and/or other suitable high-density materials for use in radiographic imaging. The marker 1000 is expected to operate in a manner similar to the marker 700 described above.

Figure 11 is an isometric view with a cut away portion of a marker 1100 in accordance with yet another embodiment of the invention. The marker 1100 is substantially similar to the markers 700 and 1000 shown in Figures 7A-C and Figure 10, and thus like reference numbers refer to like components in these Figures. The marker 1100 includes an imaging element 1130 including a first contrast element 1132 and a second contrast element 1134. The first and second contrast elements 1132/1134 can be positioned proximate to opposing ends of the magnetic transponder 120. The first and second contrast elements 1132/1134 can be discontinuous rings having a gap 1135 to mitigate eddy currents. The contrast elements 1132/1134 can be composed of the same materials as described above with respect to the contrast elements of other imaging elements in accordance with other embodiments of the invention.

Additional embodiments of markers in accordance with the invention can include imaging elements incorporated into or otherwise integrated with the casing 110, the core 728 (Figure 7B) of the magnetic transponder 120, and/or the adhesive 729 (Figure 7B) in the casing. For example, particles of a high density material can be mixed with ferrite and extruded to form the core 728. Alternative embodiments can mix particles of a high density material with glass or another material to form the casing 110, or coat the casing 110 with a high-density material. In still other embodiments, a high density material can be mixed with the adhesive 729 and injected into the casing 110. Any of these embodiments can incorporate the high density material into a combination of the casing 110, the core 728 and/or the adhesive 729. Suitable high density materials can include tungsten, gold and/or platinum as described above.

From the foregoing, it will be appreciated that specific embodiments of the invention have been described herein for purposes of illustration, but that various modifications may be made without deviating from the scope of the claims. For example, the imaging elements can be composed of more than one material, or the imaging elements of the various embodiments can be interchanged or combined with each other. Another embodiment could accordingly have the following : (a) a casing; (b) a magnetic transponder at least partially in the casing that produces a wirelessly transmitted signal in response to a wirelessly transmitted excitation energy; and (c) an imaging element including a ring-like contrast element at one end of the transponder and a spherical contrast element at the other end of the transponder. Still another embodiment can include the MRI compatible ferromagnetic element 140 described above with reference to Figures 1-6 as a core and the imaging elements described above with reference to Figures 7A-11. For example, this embodiment of the marker comprises: (a) a casing configured to be positioned at a selected location relative to a target of the patient; (b) a magnetic transponder that produces a wirelessly transmitted signal in response to a wirelessly transmitted excitation energy, wherein the magnetic transponder includes a ferromagnetic core having a volume such that when the marker is in an imaging magnetic field having a field strength of 1.5 T and a gradient of 3 T/m, then the force exerted on the marker by the imaging magnetic filed is not great than gravitational force exerted on the marker; and (c) an imaging element incorporated with the casing and/or the magnetic transponder, wherein the imaging element produces a radiographic profile in a radiographic image such that the marker has a radiographic centroid at least approximately coincident with the magnetic centroid.

## Claims

1. A leadless implantable marker (100) for localizing the position of a target within a patient, comprising:
a ferromagnetic core (140), wherein a diameter of the ferromagnetic core (140) is 0.30-0.35 mm and a length of the ferromagnetic core (140) is 4.0-6.0 mm;
a resonating circuit (120) comprising a conductive element having a plurality of windings surrounding at least a portion of the ferromagnetic core (140), wherein the resonating circuit (120) is not coupled to external electrical leads; and
a casing (110) enclosing the core (140) and the resonating circuit (120).

2. A method of tracking a target of a patient, comprising:
imaging a leadless implantable marker (100) attached to the patient, the marker (100) comprising:
a ferromagnetic core (140), wherein a diameter of the ferromagnetic core (140) is 0.30 to 0.35 mm and a length of the ferromagnetic core (140) is 4.0-6.0 mm;
a resonating circuit (120) comprising a conductive element having a plurality of windings surrounding at least a portion of the ferromagnetic core (140), wherein the resonating circuit (120) is not coupled to external electrical leads; and
a casing (110) enclosing the core (140) and the resonating circuit (120);
using a first energy to obtain an image of the marker (100), the marker (100) having a magnetic transponder that produces a wirelessly transmitted signal in response to a wirelessly transmitted excitation energy; and locating the marker (100) by transmitting the excitation energy to the marker (100).

3. The method of claim 2, wherein the first energy is radiation.

4. The method of claim 2, wherein the marker (100) further comprises an imaging element that is opaque to the radiation such that the marker has a radiographic centroid at least approximately coincident with a magnetic centroid of the magnetic transponder, and the imaging procedure comprises irradiating the marker (100).

5. A method of manufacturing a leadless implantable marker (100) for localizing the position of a target within a patient, comprising:
manufacturing a ferromagnetic element (140) having a diameter of 0.30-0.35 mm and a length of 4.0-6.0 mm;
placing a coil (122) around the ferromagnetic element (140) and encapsulating a resonating circuit (120) and the ferromagnetic element (140) in a casing (110), wherein the resonating circuit (120) comprises the coil and the coil comprises a conductive element having a plurality of windings surrounding at least a portion of the ferromagnetic element (140) and wherein the resonating circuit (120) is not coupled to external electrical leads.

6. The method of claim 5, further comprising providing a retractable sleeve that is slideable along the ferromagnetic element (140) as the conductor (124) is wound directly onto the ferromagnetic element (140).

## Patentansprüche

1. Bleifreier implantierbarer Marker (100) zum Lokalisieren der Position eines Ziels an einem Patienten, umfassend:
einen ferromagnetischen Kern (140), wobei ein Durchmesser des ferromagnetischen Kerns (140) 0,30 bis 0,35 mm und eine Länge des ferromagnetischen Kerns (140) 4,0 bis 6,0 mm beträgt;
eine Resonanzschaltung (120), umfassend ein leitfähiges Element mit einer Mehrzahl von Windungen, die wenigstens einen Abschnitt des ferromagnetischen Kerns (140) umgibt, wobei die Resonanzschaltung (120) nicht an externe elektrische Leiter gekoppelt ist; und
ein Gehäuse (110), das den Kern (140) und die Resonanzschaltung (120) umschließt.

2. Verfahren zum Verfolgen eines Ziels eines Patienten, umfassend:
Abbilden eines bleifreien implantierbaren Markers (100), der an dem Patienten angebracht ist, wobei der Marker (100) Folgendes umfasst:
einen ferromagnetischen Kern (140), wobei ein Durchmesser des ferromagnetischen Kerns (140) 0,30 bis 0,35 mm und eine Länge des ferromagnetischen Kerns (140) 4,0 bis 6,0 mm beträgt;
eine Resonanzschaltung (120), umfassend ein leitfähiges Element mit einer Mehrzahl von Windungen, die wenigstens einen Abschnitt des ferromagnetischen Kerns (140) umgibt, wobei die Resonanzschaltung (120) nicht an externe elektrische Leiter gekoppelt ist; und
ein Gehäuse (110), das den Kern (140) und die Resonanzschaltung (120) umschließt;
Verwenden einer ersten Energie zum Erlangen eines Bilds des Markers (100), wobei der Marker (100) einen magnetischen Transponder aufweist, der ein drahtlos übertragenes Signal in Reaktion auf eine drahtlos übertragene Erregungsenergie erzeugt; und
Lokalisieren des Markers (100) durch Übertragen der Erregungsenergie an den Marker (100).

3. Verfahren nach Anspruch 2, wobei die erste Energie Strahlung ist.

4. Verfahren nach Anspruch 2, wobei der Marker (100) ferner ein Bildgebungselement umfasst, das undurchlässig für die Strahlung ist, derart, dass der Marker einen radiografischen Schwerpunkt aufweist, der wenigstens annähernd mit einem magnetischen Schwerpunkt des magnetischen Transponders übereinstimmt, und der Bildgebungsvorgang Bestrahlen des Markers (100) umfasst.

5. Verfahren zum Herstellen eines bleifreien implantierbaren Markers (100) zum Lokalisieren der Position eines Ziels in einem Patienten, umfassend:
Herstellen eines ferromagnetischen Elements (140) mit einem Durchmesser von 0,30 bis 0,35 mm und einer Länge von 4,0 bis 6,0 mm;
Anordnen einer Spule (122) um das ferromagnetische Element (140) und Verkapseln einer Resonanzschaltung (120) und des ferromagnetischen Elements (140) in einem Gehäuse (110), wobei die Resonanzschaltung (120) die Spule umfasst und die Spule ein leitfähiges Element mit einer Mehrzahl von Windungen umfasst, die wenigstens einen Abschnitt des ferromagnetischen Elements (140) umgeben, und wobei die Resonanzschaltung (120) nicht an externe elektrische Leiter gekoppelt ist.

6. Verfahren nach Anspruch 5, ferner umfassend Bereitstellen einer einziehbaren Hülse, die an dem ferromagnetischen Element (140) entlang gleiten kann, wenn der Leiter (124) direkt um das ferromagnetische Element (140) gewickelt wird.

## Revendications

1. Marqueur implantable sans fil (100) pour la localisation de la position d'une cible à l'intérieur d'un patient, comprenant :
un noyau ferromagnétique (140), dans lequel un diamètre du noyau ferromagnétique (140) est de 0,30-0,35 mm et une longueur du noyau ferromagnétique (140) est de 4,0-6,0 mm ;
un circuit résonant (120) comprenant un élément conducteur ayant une pluralité d'enroulements encerclant au moins une partie du noyau ferromagnétique (140), dans lequel le circuit résonant (120) n'est pas couplé à des fils électriques externes ; et
une enveloppe (110) enfermant le noyau (140) et le circuit résonant (120).

2. Procédé de suivi d'une cible d'un patient, comprenant :
l'imagerie d'un marqueur implantable sans fil (100) fixé au patient, le marqueur (100) comprenant :
un noyau ferromagnétique (140), dans lequel un diamètre du noyau ferromagnétique (140) est de 0,30-0,35 mm et une longueur du noyau ferromagnétique (140) est de 4,0-6,0 mm ;
un circuit résonant (120) comprenant un élément conducteur ayant une pluralité d'enroulements encerclant au moins une partie du noyau ferromagnétique (140), dans lequel le circuit résonant (120) n'est pas couplé à des fils électriques externes ; et
une enveloppe (110) enfermant le noyau (140) et le circuit résonant (120) ;
l'utilisation d'une première énergie pour obtenir une image du marqueur (100), le marqueur (100) ayant un transpondeur magnétique qui produit un signal transmis sans fil en réponse à une énergie d'excitation transmise sans fil ; et
la localisation du marqueur (100) par transmission de l'énergie d'excitation au marqueur (100).

3. Procédé selon la revendication 2, dans lequel la première énergie est un rayonnement.

4. Procédé selon la revendication 2, dans lequel le marqueur (100) comprend en outre un élément d'imagerie qui est opaque au rayonnement de sorte que le marqueur a un centroïde radiographique au moins approximativement coïncidant avec un centroïde magnétique du transpondeur magnétique, et la procédure d'imagerie comprend l'irradiation du marqueur (100).

5. Procédé de fabrication d'un marqueur implantable sans fil (100) pour la localisation de la position d'une cible à l'intérieur d'un patient, comprenant :
la fabrication d'un élément ferromagnétique (140) ayant un diamètre de 0,30-0,35 mm et une longueur de 4,0-6,0 mm ;
le placement d'une bobine (122) autour de l'élément ferromagnétique (140) et l'encapsulage d'un circuit résonant (120) et de l'élément ferromagnétique (140) dans une enveloppe (110), dans lequel le circuit résonant (120) comprend la bobine et la bobine comprend un élément conducteur ayant une pluralité d'enroulements encerclant au moins une partie de l'élément ferromagnétique (140) et dans lequel le circuit résonant (120) n'est pas couplé à des fils électriques externes.

6. Procédé selon la revendication 5, comprenant en outre la fourniture d'un manchon rétractable qui peut glisser le long de l'élément ferromagnétique (140) étant donné que le conducteur (124) est enroulé directement sur l'élément ferromagnétique (140).
